# EUROPEAN PATENT APPLICATION

(11) **EP 2 853 243 A1**
(43) Date of publication of application: **01.04.2015**
(21) Application number: 13793076.4
(22) Date of filing: 26.04.2013
(51) Int. Cl.: A61F 2/82

(54) **STENT, DEVICE FOR INSERTING STENT, AND SURGICAL OPERATION METHOD USING SAME**

(30) Priority: 23.05.2012 KR 20120054741
(71) Applicant: Institute for Research & Industry Cooperation PNU, Busan 609-735 (KR); S&G Biotech, Inc, Gyeonggi-do 462-705 (KR)
(72) Inventor: LEE, Han Cheol, Busan 602-739 (KR); KANG, Sung Kwon, Yongin-si Gyeonggi-do 448-538 (KR); JUNG, So Hee, Seoul 143-824 (KR)
(74) Representative: Ter Meer Steinmeister & Partner
(86) International application number: PCT/KR2013/003624
(87) International publication number: WO 2013/176409

(57) **Abstract**

A device for inserting a stent of the present invention comprises: a first stent insertion unit; and a second stent insertion unit. The first stent insertion unit comprises: a first insertion portion formed by extending a first outer tube; a first aiding portion formed by extending a first inner tube inserted into the first insertion portion to be slid back and forth inside the first outer tube; a first central tube into which a first guide wire is to be inserted, and which is inserted into the first aiding portion to penetrate the first inner tube; and a first stent to be inserted into the front end of the first outer tube while encompassing the circumference of the first central tube and is discharged outside the first outer tube by forward movement of the first inner tube so as to be extended. The second stent insertion unit comprises: a second insertion portion formed by extending a second outer tube to be inserted into the first outer tube; a second aiding portion formed by extending a second inner tube inserted into the second insertion portion to slide back and forth inside the second outer tube; a second central tube which is inserted into the first insertion portion to be extended between the first outer tube and the first inner tube, into which a second guide wire fixed to the first stent is inserted, and which is inserted into the second aiding portion to penetrate the second inner tube; and a second stent to be inserted into the front end of the second outer tube while encompassing the circumference of the second central tube and is discharged outside the second outer tube by forward movement of the second inner tube so as to be extended. In addition, a method for inserting a stent of the present invention comprises the steps of: inserting the first guide wire into a main stenosis region; inserting the first guide wire into the first central tube to insert the first outer tube into the main stenosis region along the first guide wire; operating the first stent insertion unit to move the first inner tube forward, thereby discharging the first stent to the main stenosis region and placing the front end of the second guide wire at a branch stenosis region; expanding the first stent; retreating the first inner tube to secure an inner space of the first outer tube; inserting the back end of the second guide wire into the second central tube to insert the second outer tube into the branch stenosis region along the second guide wire; operating the second stent insertion unit to move the second inner tube forward, thereby discharging the second stent to the branch stenosis region; and expanding the second stent. Additionally, a stent of the present invention comprises an artificial blood vessel comprising: a body in which a plurality of holes are formed on the surface, a hollow is formed inside by intersecting wires, and a circumference can be extended and contracted; and a marker which encompasses the circumference of the body, has a through-hole at one side, and guides the displacement of the body.

## Description

### [Technical Field]

The present invention relates to a device for inserting a stent that allows a stent to be installed even at a branch stenosis region other than a main stenosis region, when stenosis occurs at a blood vessel in a human body, and more particularly, to a stent, a device for inserting the stent, and a surgical operation method using the same that can install the stent by inserting an insertion tube for a branch stenosis region into the branch stenosis region through an insertion tube inserted into a main blood vessel.

### [Background Art]

In general, when stenosis occurs at organs which need to secure a predetermined inner diameter, such as a blood vessel, a throat, and the like in a human body due to causes such as a deposit, and the like, a device for inserting a stent that insets a stent so as to artificially extend a stenosis region is used.

However, the device for inserting a stent in the related art has a problem that when a branch stenosis region derived from the stenosis region is adjacent, it is difficult to further insert a separate stent into the branch stenosis region. Further, when the stenosis is caused due to the deposit, and the like, a phenomenon frequently occurs, in which the deposit is pushed into the branch stenosis region while installing the stent at the main stenosis region to increase a difficulty in an insertion surgical operation of the stent.

A technology that simultaneously inserts the stent into the branch stenosis region as well as the main stenosis region has been researched in order to solve such a problem, but the technology is a technology regarding a stent, which has an extension method using a balloon catheter. Therefore, there is a need for a research into a technology that inserts an autonomous extension type stent into the branch stenosis region.

In addition, since the technology that inserts the stent into the branch stenosis region by using the balloon catheter uses a method for simultaneously inserting the main stenosis region and the branch stenosis region by segmenting a part of the stent, the technology has a problem that the surgical operation is difficult when there are two or more branch stenosis regions.

Accordingly, a method for solving the problems is required.

### [Disclosure]

### [Technical Problem]

The present invention has been made in an effort to provide a stent, a device for inserting the stent, and a surgical operation using the same that previously install second guide wires which are as many as stents to be inserted and guide tubes guiding the second guide wires into a first insertion portion.

The present invention has also been made in an effort to provide a stent, a device for inserting the stent, and a surgical operation using the same that include a second stent insertion unit including a second insertion portion and a second aiding portion in addition to a first stent insertion unit including a first insertion portion and a first aiding portion in order to install a stent for a branch stenosis region.

Technical objects to be solved by the present invention are not limited to the aforementioned technical object and other unmentioned technical objects will be clearly understood by those skilled in the art from the following description.

### [Technical Solution]

An exemplary embodiment of the present invention provides a device for inserting a stent, including: a first stent insertion unit including a first insertion portion formed by extending a first outer tube, a first aiding portion formed by extending a first inner tube inserted into the first insertion portion to be slid back and forth inside the first outer tube, a first central tube into which a first guide wire is to be inserted, and which is inserted into the first aiding portion to penetrate the first inner tube, and a first stent inserted into the front end of the first outer tube while encompassing the circumference of the first central tube and discharged outside the first outer tube by forward movement of the first inner tube so as to be extended; and a second stent insertion unit including a second insertion portion formed by extending a second outer tube to be inserted into the first outer tube, a second aiding portion formed by extending a second inner tube inserted into the second insertion portion to be slid back and forth inside the second outer tube, a second central tube which is inserted into the first insertion portion to be extended between the first outer tube and the first inner tube, into which a second guide wire fixed to the first stent is inserted, and which is inserted into the second aiding portion to penetrate the second inner tube, and a second stent inserted into the front end of the second outer tube while encompassing the circumference of the second central tube and discharged outside the second outer tube by forward movement of the second inner tube so as to be extended.

One or more extension preventing members that prevent extension of the stent may be provided on the circumference of at least one of the first stent and the second stent, and fixation wires may be further provided, which fix the extension preventing members and are extended through the corresponding inner tubes to protrude outside the corresponding aiding portions.

A guide member may be provided at the front end of at least one of the first central tube and the second central tube, in which the guide member protrudes outside each of the corresponding outer tubes and has a larger diameter than each of the corresponding outer tubes.

The second guide wire may be inserted into the inside of the first insertion portion through the rear of the first aiding portion.

The second guide wires may be provided as many as stents to be installed.

An blood vessel graft may be provided on the circumference of at least one of the first stent and the second stent, and a through-hole through which the second guide wire passes may be formed at the blood vessel graft provided in the first stent.

A fixation member that fixes the first inner tube may be provided at the first insertion portion, in a part where the first inner tube is inserted into the inside of the first insertion portion.

The device may further include a third stent insertion unit including a third insertion portion formed by extending a third outer tube inserted into the second outer tube, a third aiding portion formed by extending a third inner tube inserted into the third insertion portion to be slid back and forth inside the third outer tube, a third central tube which is inserted into the third insertion portion to be extended between the second outer tube and the second inner tube, into which a third guide wire fixed to the second stent is inserted, and which is inserted into the third aiding portion to penetrate the third inner tube, and a third stent inserted into the front end of the third outer tube while encompassing the circumference of the third central tube and discharged outside the third outer tube by forward movement of the third inner tube so as to be extended.

Another exemplary embodiment of the present invention provides a device for inserting a stent, including: a first stent insertion unit including a first insertion portion formed by extending a first outer tube, a first aiding portion formed by extending a first inner tube inserted into the first insertion portion to be slid back and forth inside the first outer tube, a first central tube into which a first guide wire is to be inserted, and which is inserted into the first aiding portion to penetrate the first inner tube, and a first stent inserted into the front end of the first outer tube while encompassing the circumference of the first central tube and discharged outside the first outer tube by forward movement of the first inner tube so as to be extended; and a second stent insertion unit including a second insertion portion formed by extending a second outer tube to be inserted into the first outer tube, a second aiding portion formed by extending a second inner tube inserted into the second insertion portion to be slid back and forth inside the second outer tube, a second central tube which is inserted into the first insertion portion to be extended between the first outer tube and the first inner tube, into which a guide tube fixed to the first stent is inserted so as to insert a second guide wire, and which is inserted into the second aiding portion to penetrate the second inner tube, and a second stent inserted into the front end of the second outer tube while encompassing the circumference of the second central tube and discharged outside the second outer tube by forward movement of the second inner tube so as to be extended.

One or more extension preventing members that prevent extension of the stent may be provided on the circumference of at least one of the first stent and the second stent, and fixation wires may be further provided, which fix the extension preventing members and are extended through the corresponding inner tubes to protrude outside the corresponding aiding portions.

A guide member may be provided at the front end of at least one of the first central tube and the second central tube, in which the guide member protrudes outside each of the corresponding outer tubes and has a larger diameter than each of the corresponding outer tubes.

The guide tube may be inserted into the inside of the first insertion portion through the rear of the first aiding portion.

The guide tubes may be provided as many as stents to be installed.

An blood vessel graft may be provided on the circumference of at least one of the first stent and the second stent, and a through-hole through which the guide tube passes may be formed at the blood vessel graft provided in the first stent.

A fixation member that fixes the first inner tube may be provided at the first insertion portion, in a part where the first inner tube is inserted into the inside of the first insertion portion.

Yet another exemplary embodiment of the present invention provides a surgical operation method for inserting a stent in the device for inserting a stent, including: inserting a first guide wire into a main stenosis region; inserting the first guide wire into a first central tube to insert a first outer tube into the main stenosis region along the first guide wire; operating a first stent insertion unit to move the first inner tube forward, thereby discharging a first stent to the main stenosis region and placing the front end of a second guide wire at a branch stenosis region; retreating a first inner tube to secure an inner space of the first outer tube; inserting the back end of the second guide wire into a second central tube to insert a second outer tube into the branch stenosis region along the second guide wire; operating the second stent insertion unit to move the second inner tube forward, thereby discharging the second stent to the branch stenosis region; and extending the second stent.

One or more extension preventing members that prevent extension of the stent may be provided on the circumference of at least one of the first stent and the second stent and fixation wires may be provided, which fix the extension preventing members and are extended through each corresponding inner tube to protrude outside each corresponding aiding portion, and at least one of the extending of the first stent and the extending of the second stent may include removing the fixation wire from the extension preventing member.

A plurality of second guide wires may be provided, and after the extending of the second stent, retreating the second outer tube to secure an inner space of the first outer tube, inserting the rear end of another second guide wire into the second stent insertion unit or a second central tube of another second stent insertion unit to insert a second outer tube into another branch stenosis region along another second guide wire, operating the second stent insertion unit to move the second inner tube forward, thereby discharging another second stent to another branch stenosis region, and extending another second stent may be repeatedly performed as many as the second guide wires.

A fixation member that fixes the first inner tube may be provided at the first insertion portion, in a part where the first inner tube is inserted into the inside of the first insertion portion, and fixing the first inner tube with the fixation member may be further included between the securing of the inner space of the first outer tube and the inserting of the second outer tube into the branch stenosis region.

Still another exemplary embodiment of the present invention provides a surgical operation method for inserting a stent in the device for inserting a stent, including: inserting a first guide wire into a main stenosis region; inserting the first guide wire into a first central tube to insert a first outer tube into the main stenosis region along the first guide wire; operating a first stent insertion unit to move the first inner tube forward, thereby discharging a first stent to the main stenosis region and placing the front end of a second guide wire at a branch stenosis region; extending the first stent; inserting the second guide wire into the guide tube; retreating the first inner tube and the guide wire to secure an inner space of the first outer tube; inserting the back end of the second guide wire into a second central tube to insert a second outer tube into the branch stenosis region along the second guide wire; operating the second stent insertion unit to move the second inner tube forward, thereby discharging the second stent to the branch stenosis region; and extending the second stent.

One or more extension preventing members that prevent extension of the stent may be provided on the circumference of at least one of the first stent and the second stent and fixation wires may be provided, which fix the extension preventing members and are extended through each inner tube to protrude outside each corresponding aiding portion, and at least one of the extending of the first stent and the extending of the second stent may include removing the fixation wire from the extension preventing member.

A fixation member that fixes the first inner tube may be provided at the first insertion portion, in a part where the first inner tube is inserted into the inside of the first insertion portion, and fixing the first inner tube with the fixation member may be further included between the securing of the inner space of the first outer tube and the inserting of the second outer tube into the branch stenosis region.

Still yet another exemplary embodiment of the present invention provides a stent including: a body in which a plurality of holes are formed on the surface, a hollow is formed inside by intersecting wires, and a circumference is extensible and contractable; and an blood vessel graft provided to encompass the circumference of the body, has a through-hole at one side, and guides the displacement of the body.

The marker may include a first marker formed on the circumference of the through-hole, a third marker formed on the opposite circumference of the through-hole, and a second marker including a reference marker formed between the first marker and the third marker, and the reference and a comparison marker formed between the first marker and the third marker and allowing a rotational direction of the body to be recognized through comparison with each other.

### [Advantageous Effects]

According to exemplary embodiments of the present invention, a stent, a device for inserting the stent, and a surgical operation using the same acquire the following effects.

First, since second guide wires which are as many as stents to be inserted or guide tubes guiding the second guide wires are used, it is possible to easily insert each stent by positioning the second guide wires or the guide tubes for each branch stenosis region even when two or more branch stenosis regions are present.

Second, even when another branch stenosis region is present at the branch stenosis region, it is possible to insert the stent even into another branch stenosis region by a method for previously installing the stent even into a second insertion portion like a first insertion portion.

Third, since a second stent insertion unit including the second insertion portion and a second aiding portion is used in order to install a stent for a branch stenosis region, there is no need to separately operate a first outer tube positioned at a main stenosis region.

Fourth, since a first stent insertion unit can be formed to have the same shape as that of the second stent insertion unit, there is no need to learn a separate operation and an overall operation can be easy.

The effects of the present invention are not limited to the aforementioned effects, and other effects, which are not mentioned above, will be apparent to those skilled in the art from the description included in the appended claims

### [Description of Drawings]

FIG. 1 is a side view illustrating an overall shape of a device for inserting a stent according to a first exemplary embodiment of the present invention;
FIG. 2 is a perspective view illustrating the front end of a first outer tube in the device for inserting a stent according to the first exemplary embodiment of the present invention;
FIG. 3 is a cross-sectional view illustrating the inside of the front end of the first outer tube in the device for inserting a stent according to the first exemplary embodiment of the present invention;
FIG. 4 is a cross-sectional view illustrating a state in which a first guide wire is, in advance, inserted into a main stenosis region in the device for inserting a stent according to the first exemplary embodiment of the present invention;
FIG. 5 is a cross-sectional view illustrating a state in which the first outer tube is inserted into the main stenosis region along the first guide wire in the device for inserting a stent according to the first exemplary embodiment of the present invention;
FIG. 6 is a cross-sectional view illustrating a state in which a part of a first stent is discharged by moving a first inner tube forward in the device for inserting a stent according to the first exemplary embodiment of the present invention;
FIG. 7 is a cross-sectional view illustrating a state in which the first stent is extended to the main stenosis region by removing a fixation wire in the device for inserting a stent according to the first exemplary embodiment of the present invention;
FIG. 8 is a cross-sectional view illustrating a state in which an inner space of the first outer tube is secured by retreating the first inner tube in the device for inserting a stent according to the first exemplary embodiment of the present invention;
FIG. 9 is a cross-sectional view illustrating a state in which a second outer tube is inserted along a second guide wire in the device for inserting a stent according to the first exemplary embodiment of the present invention;
FIG. 10 is a cross-sectional view illustrating a state in which the second outer tube is positioned at a branch stenosis region in the device for inserting a stent according to the first exemplary embodiment of the present invention;
FIG. 11 is a cross-sectional view illustrating a state in which a second stent is discharged by moving a second inner tube forward in the device for inserting a stent according to the first exemplary embodiment of the present invention;
FIG. 12 is a cross-sectional view illustrating a state in which the second stent is extended to the branch stenosis region by removing the fixation wire in the device for inserting a stent according to the first exemplary embodiment of the present invention;
FIG. 13 is a cross-sectional view illustrating a state in which the device for inserting a stent is removed after installing each stent in the device for inserting a stent according to the first exemplary embodiment of the present invention;
FIG. 14 is a perspective view illustrating the front end of a first outer tube in a device for inserting a stent according to a second exemplary embodiment of the present invention;
FIG. 15 is a cross-sectional view illustrating a state in which a part of a first stent is discharged by moving a first inner tube forward in the device for inserting a stent according to the second exemplary embodiment of the present invention;
FIG. 16 is a cross-sectional view illustrating a state in which a second guide wire is inserted along a guide tube in the device for inserting a stent according to the second exemplary embodiment of the present invention;
FIG. 17 is a side view illustrating an overall shape of a device for inserting a stent according to a third exemplary embodiment of the present invention;
FIG. 18 is a cross-sectional view illustrating a state in which the device for inserting a stent is removed after installing each stent in the device for inserting a stent according to the third exemplary embodiment of the present invention;
FIG. 19 is a side view illustrating an overall shape of a device for inserting a stent according to a fourth exemplary embodiment of the present invention;
FIG. 20 is a cross-sectional view illustrating a state in which the device for inserting a stent is removed after installing each stent in the device for inserting a stent according to the fourth exemplary embodiment of the present invention;
FIG. 21 is a side view illustrating the shape of a first stent in a device for inserting a stent according to a fifth exemplary embodiment of the present invention;
FIG. 22 is a side view illustrating the shape of a first stent in a device for inserting a stent according to a sixth exemplary embodiment of the present invention;
FIG. 23 is a perspective view illustrating the shape of a first stent in a device for inserting a stent according to a seventh exemplary embodiment of the present invention; and
FIG. 24 is a diagram illustrating a state in which the first stent is installed at an ascending aorta in the device for inserting a stent according to the seventh exemplary embodiment of the present invention.

### [Best Mode]

Hereinafter, exemplary embodiments of the present invention will be described in detail with reference to the accompanying drawings. However, in describing the present invention, a detailed description of already known functions or configurations will be omitted so as to make the subject matter of the present invention clear.

A device for inserting a stent according to the present invention includes a first stent insertion unit and a second stent insertion unit, and each of the first stent insertion unit and the second stent insertion unit includes an insertion portion and an aiding portion.

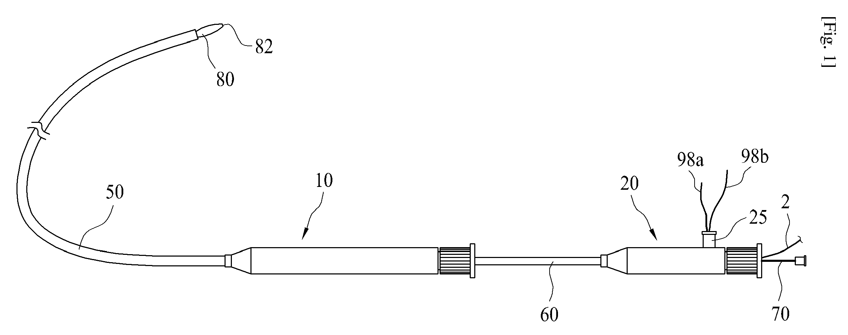

First, referring to FIG. 1, an overall shape of a device for inserting a stent according to a first exemplary embodiment of the present invention is illustrated. In FIG. 1, only the first stent insertion unit is illustrated and the second stent insertion unit will be described below with reference to another drawing.

As illustrated in FIG. 1, the first stent insertion unit includes a first insertion portion 10 and a first aiding portion 20 and a first outer tube 50 extends to the first insertion portion 10 and the a first inner tube 60 extends to the first aiding portion 20. In addition, the first inner tube 60 is inserted into the inside of the first insertion portion 10 to be inserted into an inner space of the first outer tube 50.

In addition, by operating the first insertion portion 10 and the first aiding portion 20, the first inner tube 60 is slid forward and backward in the first outer tube 50. In detail, when the first insertion portion 10 and the first aiding portion 20 are operated to decrease a distance therebetween, the first inner tube 60 moves forward in the first outer tube 50 and when the first insertion portion 10 and the first aiding portion 20 are operated to increase the distance therebetween, the first inner tube 60 moves backward in the first outer tube 50.

Meanwhile, the first insertion portion 10 and the first aiding portion 20 may have various shapes, but have bodies that elongate so as to be easily held with a hand in the first exemplary embodiment. Further, the first outer tube 50 and the first inner tube 60 extend from the fronts of the first insertion portion 10 and the first aiding portion 20 and the first inner tube 60 is inserted into the rear of the first insertion portion 10.

Moreover, a second guide wire 2 is inserted into the inside of the first insertion portion 10 in addition to the first inner tube 60 and a first central tube 70 is inserted into the first aiding portion 20 to penetrate an inner space of the first inner tube 60. This will be described below.

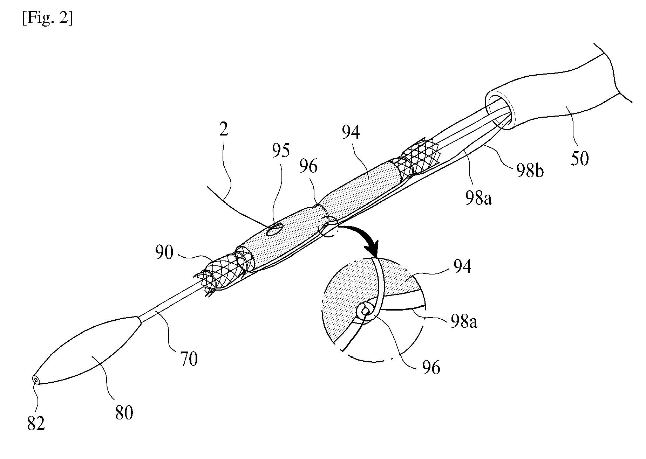

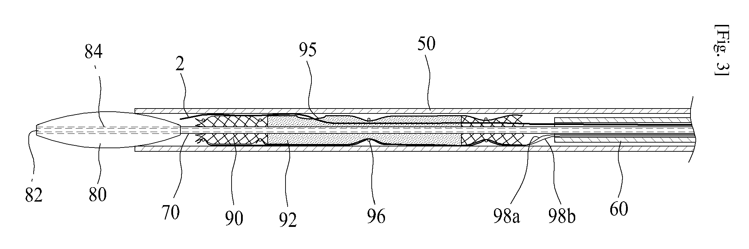

Hereinafter, the first stent insertion unit will be described with reference to FIGS. 2 and 3 for a more detailed description. In FIG. 2, the shape of a front end of the first outer tube 50 is illustrated and in FIG. 3, a cross-sectional view of the front end of the first outer tube 50 is illustrated.

First, referring to the cross-sectional view of FIG. 3, it can be seen as described above that the first inner tube 60 is inserted into the inner space of the first outer tube 50 and the first central tube 70 penetrates the inner space of the first inner tube 60.

In addition, a guide member 80 is provided at a front end of the first central tube 70 and thereafter, serves to guide a path of the first outer tube 50 by inserting a first guide wire into a guide hole 82 of an inlet. Further, a connection hole 84 connected with the guide hole 82 may be formed in the guide member 80 to be connected with an inner space of the first central tube 70 or the first central tube 70 may be inserted into the inside of the guide member 80.

In the first exemplary embodiment, the first central tube 70 is inserted into the inside of the guide member 80 as illustrated in FIG. 3. Consequently, the first guide wire may be inserted from the guide hole 82 of the guide member 80 into the inner space of the first central tube 70.

Herein, the thickness of the guide member 80 is larger than the diameter of the first outer tube 50, and as a result, the guide member 80 is not inserted into the inside of the first outer tube 50 but protrudes outward. This causes resistance generated by a body fluid to be reduced when the first outer tube 50 is inserted into a human body later and the guide member 80 to be fixed to the front end of the first outer tube 50 to play a role thereof.

Meanwhile, the guide member 80 and the first inner tube 60 are positioned to be spaced apart from each other with a predetermined distance to secure a space capable of accommodating a first stent 90 therebetween. In detail, the first stent 90 is positioned in the space between the guide member 80 and the first inner tube 60 which are spaced apart from each other while encompassing the circumference of the first central tube 70.

As a result, when the first insertion portion 10 and the first aiding portion 20 are operated to decrease the distance therebetween, the first inner tube 60 moves forward in the first outer tube 50 to push out the first stent 90. That is, the first inner tube 60 moves forward to push out the first stent 90 and the guide member 80 to the outside of the first outer tube 50, thereby discharging the first stent 90.

When the first stent 90 is discharged from the first outer tube 50 as described above, the diameter is expanded to support an inner wall of a portion where stenosis occurs and secure a predetermined diameter or more of the stenosis region. In this case, the first stent 90 may be expanded by other devices including a balloon catheter, and the like or autonomously expanded.

In the first exemplary embodiment, the first stent 90 is autonomously expanded and this may be implemented by a method in which the first stent 90 is manufactured by a shape memory alloy or a method in which the first stent 90 is manufactured to have elasticity.

Herein, in the first exemplary embodiment, the portion where the stenosis occurs is a blood vessel, and as a result, an blood vessel graft 94 may be provided to encompass the outside of the first stent 90. Since the blood vessel graft 94 is known and commonly used art, a detailed description thereof will be omitted.

In addition, one or more extension preventing members 96 that prevent extension of the first stent 90 may be provided on the circumference of the first stent 90. The extension preventing members 96 serve to prevent the extension of the first stent 90 even when the first stent 90 is discharged outside the first outer tube 50.

When the extension preventing members 96 are not provided, the extension starts at the moment when the first stent 90 is discharged from the first outer tube 50 and when the first stent 90 is completely discharged, the first stent 90 contacts the inner wall of the stenosis region, and as a result, it is difficult to correct an installation position. On the contrary, when the extension preventing members 96 are provided, it is advantageous that the first stent 90 is discharged, and then an extension operation may be performed after controlling a minute installation position.

In detail, referring to an enlarged diagram illustrated in FIG. 2, it can be seen that the extension preventing members 96 of the first exemplary embodiment are formed in a ring shape, but are manufactured so as for elastic force to be applied in a direction in which a ring is unfolded and the bottoms are not connected to each other and have a hook shape. In this case, fixation wires 98a and 98b are further provided, which are inserted in the hook shape to fix the extension preventing members 96 so as not to be stretched.

Further, in the first exemplary embodiment, the plurality of extension preventing members 96 are provided and in particular, a total of two extension preventing members 96 are provided ahead of the blood vessel graft 94. In addition, the second fixation wire 98b is inserted into the extension preventing member close to the blood vessel graft 94 among the extension preventing members positioned ahead of the blood vessel graft 94 and the first fixation wire 98a is inserted into residual extension preventing members. That is, the extension preventing member close to the blood vessel graft 94 among the extension preventing members positioned ahead of the blood vessel graft 94 is extensible separately from other extension preventing members. A detailed description thereof will be made below.

Meanwhile, as illustrated in FIG. 1, the fixation wires 98a and 98b are extended to protrude outside the first aiding portion 20 through the first inner tube 60. That is, a protrusion member 25 illustrated in FIG. 1 is provided so as to expose the fixation wires 98a and 98b and when the fixation wires 98a and 98b exposed through the protrusion member 25 are held and extracted from the first aiding portion 20, the fixation wires 98a and 98b move backward and are thus separated from each extension preventing member 96 illustrated in FIG. 2 to extend the first stent 90. That is, the extension preventing member 96 and the fixation wires 98a and 98b interwork with each other to serve to delay the extension of the first stent 90, and as a result, a user may perform a more precise surgical operation.

In addition, the extension preventing member 96 may, of course, prevent the extension of the first stent 90 by various structures and schemes in addition to the method used in the first exemplary embodiment.

Meanwhile, the second guide wire 2 is inserted into the inside of the first insertion portion 10 and the second guide wire 2 is extended between the first outer tube 50 and the first inner tube 60, as illustrated in FIG. 1. In addition, an opposite side is fixed to the first stent 90 as illustrated in FIG. 2. That is, the second guide wire 2 may be extracted outside the first outer tube 50 together with the first stent 90 when the first stent 90 is extracted from the first outer tube 50.

Herein, when the blood vessel graft 94 is not provided in the first stent 90, the second guide wire 2 may be directly fixed to the first stent 90, but when the blood vessel graft 94 is provided as illustrated in FIG. 2, a through-hole 95 is formed in the blood vessel graft, and as a result, the second guide wire 2 passes through the through-hole 95 to be fixed.

Further, in the first exemplary embodiment, the second guide wire 2 is inserted into the rear of the first aiding portion 20 as illustrated in FIG. 1, but the present invention is not limited thereto and the second guide wire 2 may, of course, be inserted from various positions.

Hereinabove, the description of the first stent insertion unit is completed and hereinafter, a surgical operation method of the portion where the stenosis occurs in the human body will be described in detail. In addition, the portion where the stenosis occurs is set in such a manner that in addition to a main stenosis region, a branch stenosis region branched from the main stenosis region is further formed.

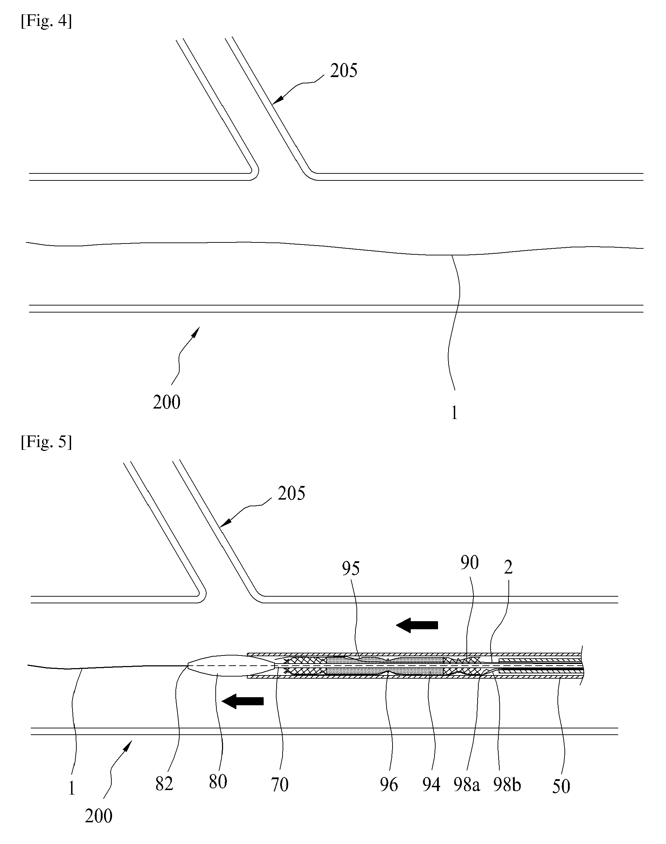

First, the first guide wire is inserted into the main stenosis region for the surgical operation. In this step, as a preceding process for inserting the device for inserting a stent into the main stenosis region, the first guide wire is inserted into the main stenosis region in advance. Referring to FIG. 4, a main stenosis region 200 and a branch stenosis region 205 are illustrated and it can be seen that the first guide wire 1 is inserted into the main stenosis region 200.

Next, as illustrated in FIG. 5, a portion of the first guide wire 1, which is exposed to the outside is inserted into the central tube 70 of the first stent insertion unit to position the first outer tube 50 at the main stenosis region 200 along the first guide wire 1. As a result, it can be seen that the front end of the first outer tube 50 is positioned at the main stenosis region 200 along the path of the first guide wire 1.

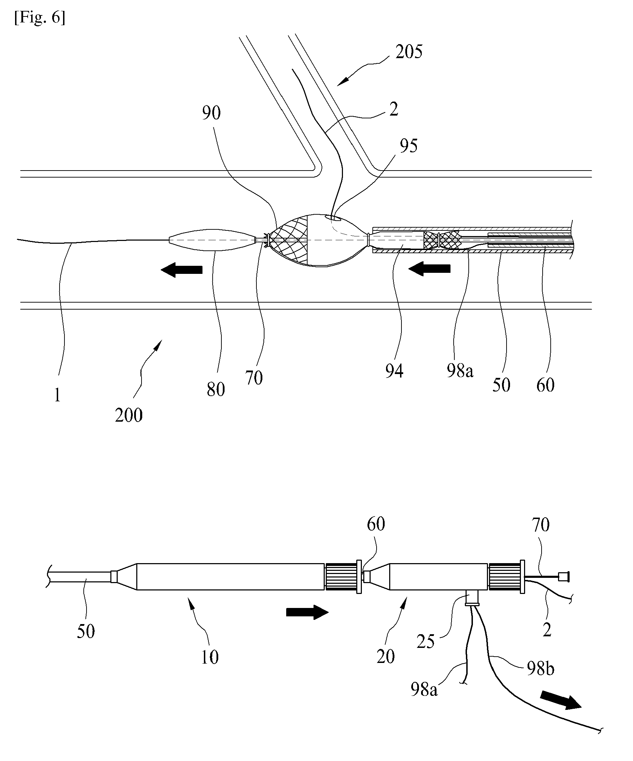

Hereinafter, as illustrated in FIG. 6, the first stent insertion unit is operated to discharge the first stent 90 from the first inner tube 60. In detail, in this step, the first insertion portion 10 and the first aiding portion 20 are operated so as to decrease the distance therebetween and the first inner tube 60 thus moves forward in the first outer tube 50, thereby pushing out the first stent 90.

Herein, for such an operation, the first aiding portion 20 may be moved forward while the first insertion portion 10 is fixed or the first insertion portion 10 may be moved backward while the first aiding portion 20 is fixed. That is, an operation for discharging the first stent 99 is performed by relative movement of the first insertion portion 10 and the first aiding portion 20 and may be performed for convenience.

However, in order to accurately set the position of the first stent 90, it is preferable that the first outer tube 50 is retreated by moving the first insertion portion 10 backward so that the position of the first stent 90 is not changed from an initial position.

Meanwhile, since the through-hole 95 is flatly transformed while the first stent 90 is provided inside the first outer tube 50, it is difficult to accurately recognize the position of the through-hole 95. That is, when the first stent 90 is completely discharged while the position of the through-hole 95 is not verified during this process, it may be difficult to position the second guide wire 2 at the branch stenosis region 205.

Accordingly, in this case, a method may be used, in which after only a part of the first stent 90 is discharged, the second fixation wire 98b is extracted from the first aiding portion 20 and only the part of the through-hole 95 is thus extended to sequentially completely discharge the first stent 90 after verifying the position of the through-hole 95 and positioning the first stent 90 at the branch stenosis region 205. The method may be used because the second fixation wire 98b is inserted into only the extension preventing member close to the blood vessel graft 94 among the extension preventing members positioned ahead of the blood vessel graft 94 as described above.

After a part of the first stent 90 is discharged through the process, the second guide wire 2 fixed to the first stent 90 is positioned at the branch stenosis region 205. In general, since the branch stenosis region 205 has a smaller diameter than the main stenosis region 200, it may be difficult to insert the second guide wire 2 into the branch stenosis region 205 by a separate operation.

However, when the second guide wire 2 is fixed to the first stent 90 in advance like the present invention, the second guide wire 2 may be easily positioned at the branch stenosis region 205 together with the operation of discharging the first stent 90 from the first outer tube 50.

Further, when the extension preventing member 96 is provided in the first stent 90, since the first stent 90 is not extended even though the first stent 90 is completely discharged from the first outer tube 50, such an operation may be more easily performed. Moreover, in this step, it is preferable that the second guide wire 2 is pushed to be further inserted inside and a subsequent operation is stably performed.

Next, the first stent 90 positioned at the main stenosis region 200 is extended. In this step, the first stent 90 is extended to secure the diameter of the inner tube of the main stenosis region 200.

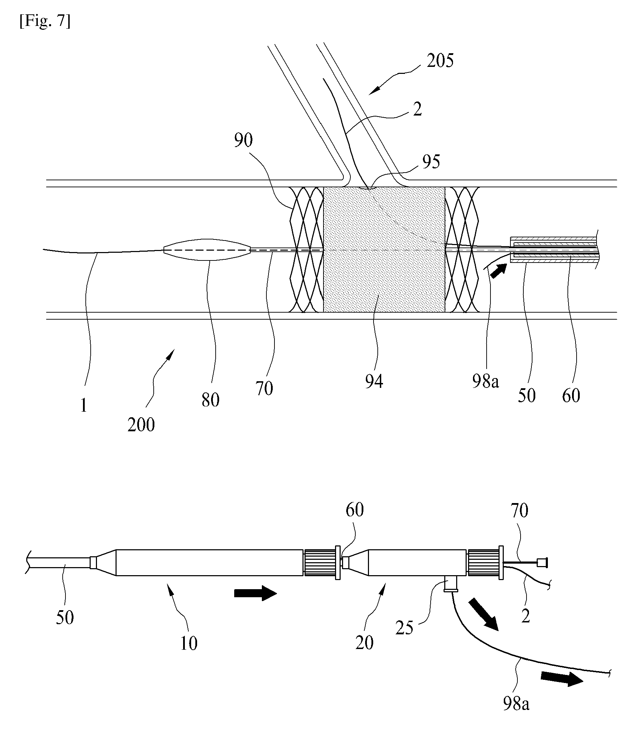

Herein, when the extension preventing member 96 is not provided in the first stent 90, the first stent 90 is autonomously extended together with the process of discharging the first stent 90 from the first outer tube 50, and as a result, this operation may be performed. Further, when the extension preventing member 96 is provided, the first stent 90 may be extended by removing the first fixation wire 98a. Referring to FIG. 7, it can be seen that the user draws out the first fixation wire 98a to completely extend the first stent 90 in the case of the first exemplary embodiment.

Even besides, when the first stent 90 is extended by the balloon catheter, this step may be performed by inflating the balloon catheter.

Through the operation up to the described step, installation of the first stent 90 at the main stenosis region 200 is completed and thereafter, an operation for installing a second stent at the branch stenosis region 205 is performed.

In order to install the second stent, the first inner tube 60 is then retreated to secure an inner space of the first outer tube 50. The reason thereforis to insert a second outer tube through the first outer tube 50 then and this will be described in a subsequent step.

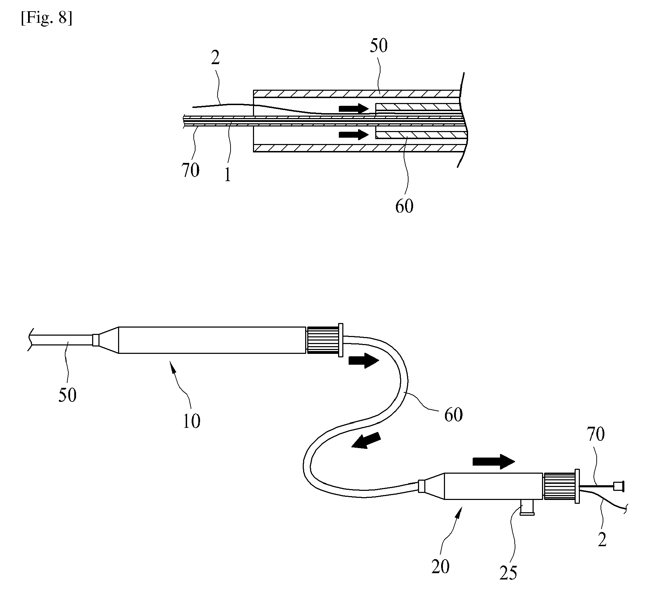

As illustrated in FIG. 8, the operation of retreating the first inner tube 60 may be performed through an operation of drawing the first aiding portion 20 to be distant from the first insertion portion 10. That is, with movement of the first aiding portion 20, the first inner tube 60 is slid backward in the first outer tube 50.

In addition, by the process, only the central tube 70 and the second guide wire 2 remain in the inner space of the first outer tube 50. That is, it can be seen that the first inner tube 60 is completely removed backward of the first insertion portion 10 to secure the inner space of the first outer tube 50.

Meanwhile, a fixation member 12 may be provided at a portion where the first inner tube 60 is inserted into the inside of the first insertion portion 10. That is, in the case of the first exemplary embodiment, since the first inner tube 60 is inserted through the rear end of the first insertion portion 10, the fixation member 12 is provided at the rear end of the first insertion portion 10. The fixation member 12 serves to control the first inner tube 60 not to move and forms a fastening structure by rotation to fix or release the fixation of the first inner tube 60 in the first exemplary embodiment.

Moreover, the fixation member may be formed at the first aiding portion 20 as well as the first insertion portion 10 and hereinafter, the fixation member may also be formed at other components having the same shape as the first insertion portion 10 and the first aiding portion 20. Therefore, a description thereof will be hereinafter omitted.

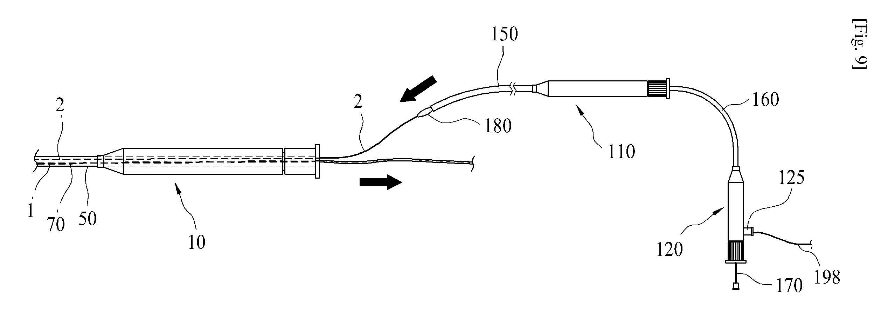

Next, as illustrated in FIG. 9, the second outer tube 150 of the second stent insertion unit is inserted into the first outer tube 50 through the rear end of the second guide wire 2 exposed through the rear of the first insertion portion 10. Herein, the second stent insertion unit is used to install the second stent at the branch stenosis region, and an overall configuration and an operation method are similar to the first stent insertion unit.

That is, the second stent insertion unit is the same as the first stent insertion unit even in that the second stent insertion unit has a second insertion portion 110 and a second aiding portion 120, and the second inner tube 160 is inserted into the second outer tube 150 and a second central tube 180 penetrates the second inner tube 160. Further, other detailed components may also be formed similarly to the first stent insertion unit.

However, in the case of the first exemplary embodiment, another guide wire is not inserted into the second insertion portion 110. Therefore, only one fixation wire 198 may be inserted into extension preventing members of the second stent unlike the case of the first stent 90.

In addition, the second stent insertion unit having a form in which another guide wire is inserted will be described below in another exemplary embodiment.

Further, since the second outer tube 150 needs to be inserted into the first outer tube 50, the second outer tube 150 has a smaller diameter than the first outer tube 50.

Consequently, the second guide wire 2 is inserted into the second central tube 170 and the second outer tube 150 is pushed into the first insertion portion 10 along the second guide wire 2 to position the second outer tube 150 at the branch stenosis region 205. That is, since the second guide wire 2 is inserted into the branch stenosis region 205 by the previous operation, the second outer tube 150 will be positioned at the branch stenosis region 205 along the second guide wire 2.

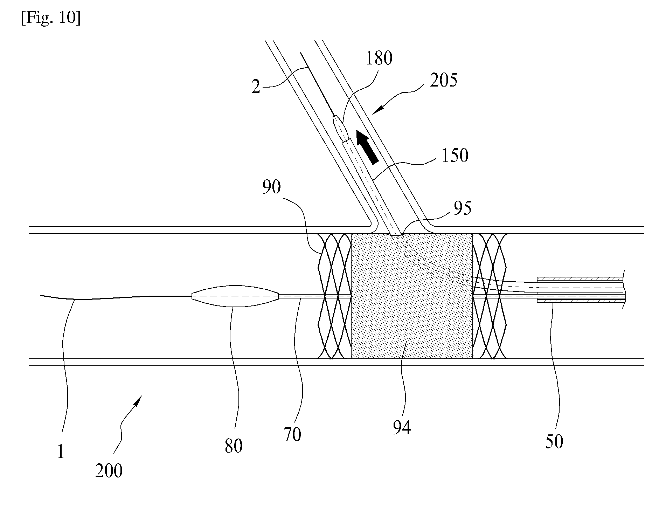

Referring to FIG. 10, it can be seen that the second outer tube 150 penetrates the first outer tube 50 through such a process and the second outer tube 150 is positioned at the branch stenosis region 205 through the through-hole 95 formed at the blood vessel graft 94 of the first stent 90.

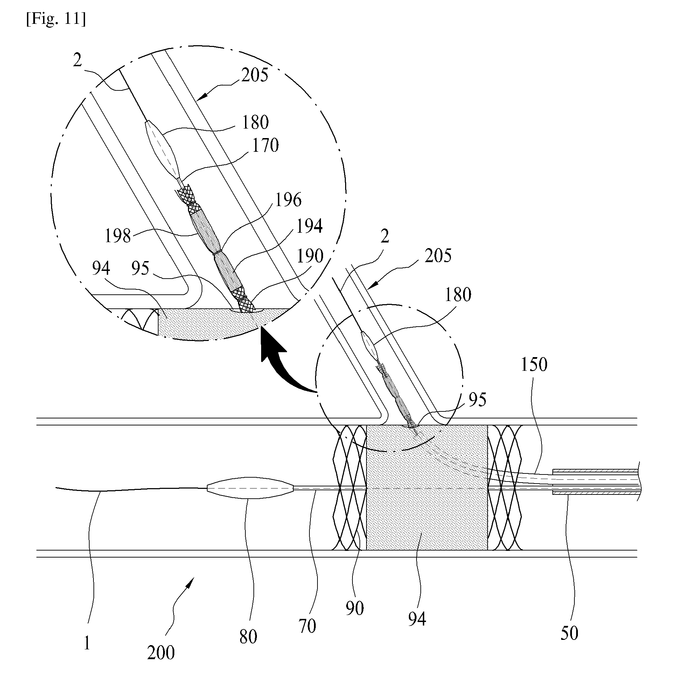

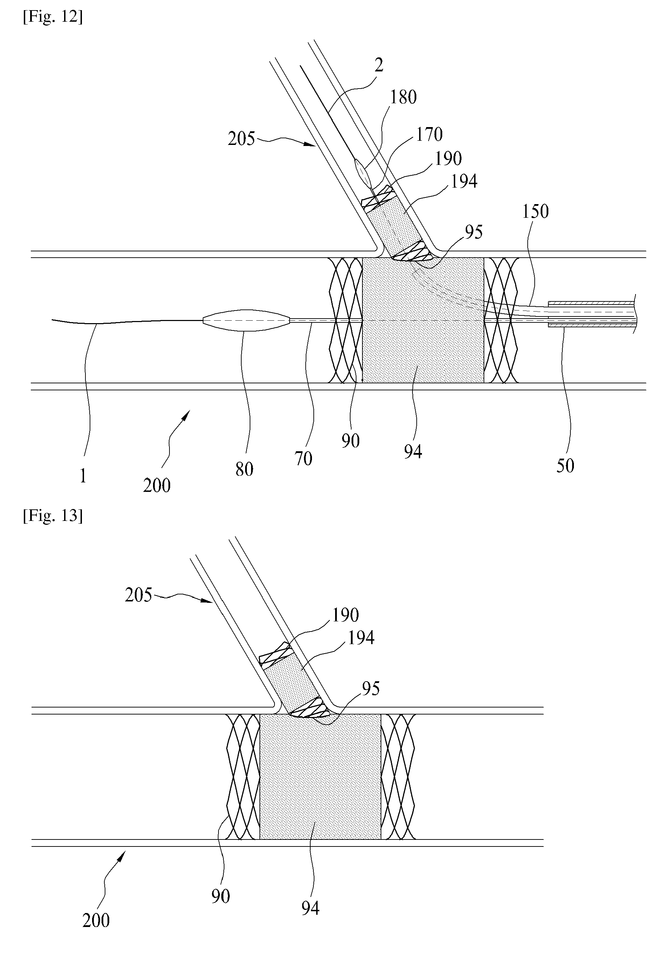

Hereinafter, a process of extending the second stent 190 is performed through the same order as the process of extending the first stent 90. That is, the second inner tube 160 illustrated in FIG. 9 moves forward by operating the second stent insertion unit to discharge the second stent 190 and the fixation wire 198 is removed to install the second stent 190 at the branch stenosis region 205. Such a process is illustrated in FIGS. 11 and 12.

Through all of the processes, installation of the first stent 90 and the second stent 190 at the main stenosis region 200 and the branch stenosis region 205, respectively is completed. Thereafter, residual components other than the respective installed stents 90 and 190 are drawn out, and as a result, the surgical operation is ended.

As described above, in the case of the first exemplary embodiment, the respective stents 90 and 190 may be easily installed at the main stenosis region 200 and the branch stenosis region 205 as illustrated in FIG. 13.

In this case, in the first exemplary embodiment, the second guide wire 2 is fixed to the first stent 90 and discharged with the discharge of the first stent 90 to be positioned at the branch stenosis region 205, but since the guide wire is generally formed with a small thickness, stiffness deteriorates. That is, an operation of positioning the second guide wire 2 at the branch stenosis region 205 according to an operation by the user may not be easy.

Accordingly, in a second exemplary embodiment of the present invention, a device for inserting a stent for solving the problem will be described.

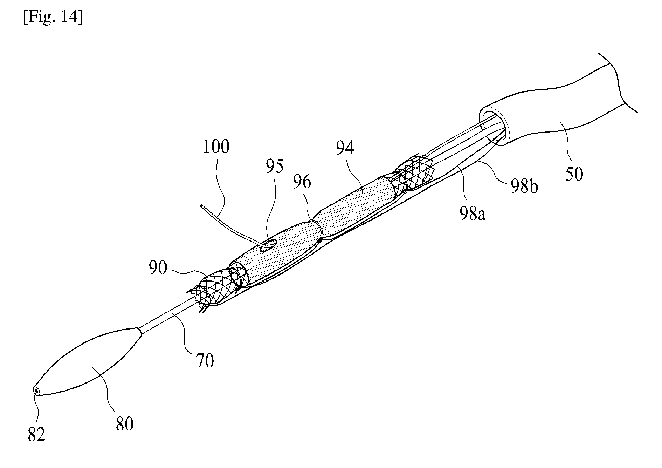

Referring to FIG. 14, in the device for inserting a stent according to the second exemplary embodiment of the present invention, the front end of the first outer tube 50 is illustrated.

The device for inserting a stent according to the second exemplary embodiment is the same as the device for inserting a stent according to the first exemplary embodiment in terms of all components. However, the devices are different from each other in that the second guide wire 2 is fixed to the first stent 90 in the first exemplary embodiment while a guide tube 100 instead of the second guide wire 2 is fixed to the first stent 90 in the second exemplary embodiment.

In detail, the guide tube 100 is a component that allows the second guide wire 2 to be thereafter inserted and is formed to have higher stiffness than the second guide wire 2. Accordingly, the guide tube 100 is more easily controlled than the second guide wire 2 to be rapidly and stably positioned at the branch stenosis region 205.

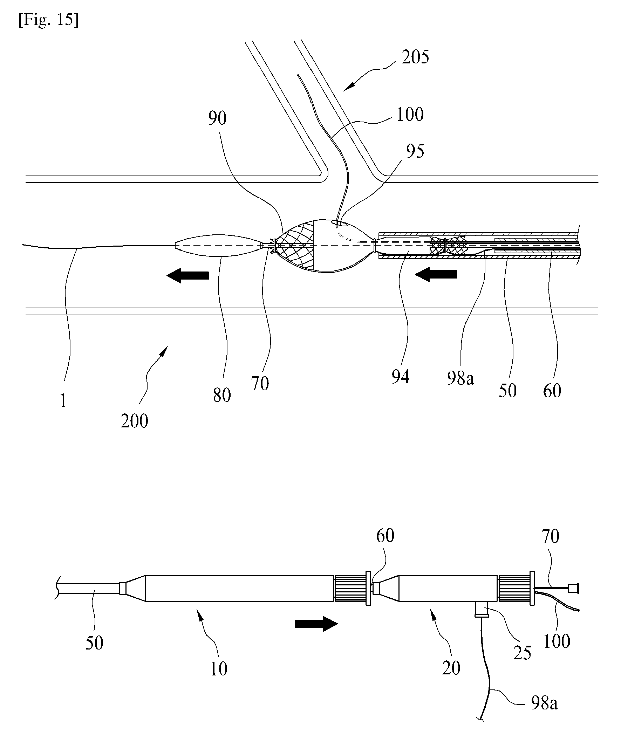

Referring to FIG. 15, it can be seen that the first insertion portion 10 and the first aiding portion 20 are operated so as to decrease the distance therebetween and the first inner tube 60 thus moves forward in the outer tube 50, thereby pushing out the first stent 90 and positioning the guide tube 100 at the branch stenosis region 205.

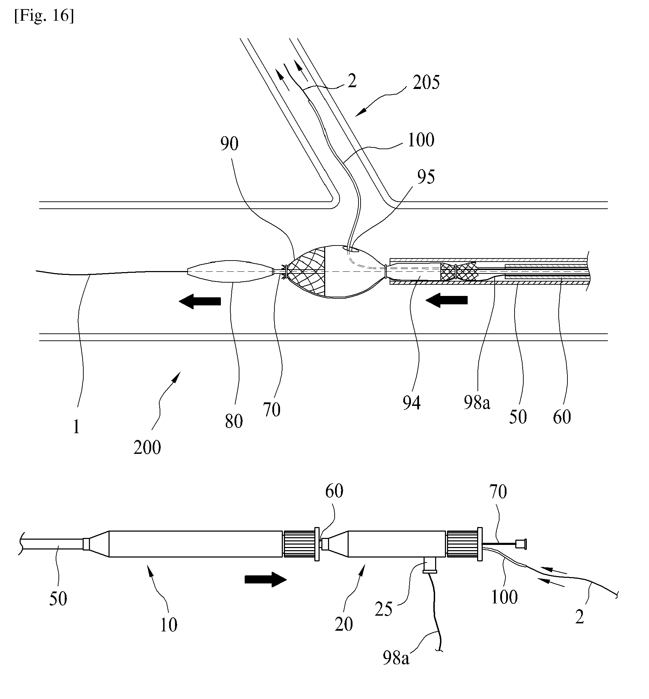

Next, referring to FIG. 16, the second guide wire 2 is inserted through the rear of the guide tube 100. That is, the second guide wire 2 may be easily positioned at the branch stenosis region 205 through the guide tube 100. Thereafter, when the guide tube 100 is together retreated and removed with the operation of securing the inner space of the first outer tube 50 by retreating the first inner tube 60, only the second guide wire 2 remains at the branch stenosis region 205.

Accordingly, thereafter, an operation of inserting the second outer tube of the second stent insertion unit into the first outer tube 50 through the rear end of the second guide wire 2 is performed similarly to the first exemplary embodiment and since a subsequent operation is similar to that of the first exemplary embodiment, a detailed description thereof will be omitted.

Hereinabove, the first exemplary embodiment and the second exemplary embodiment of the present invention have been described. In addition, the device for inserting a stent according to the present invention may be applied even to the case in which two or more branch stenosis regions 205 are present and the case in which another branch stenosis region is present at the branch stenosis region 205. A description thereof will be continued in third and fourth exemplary embodiments below.

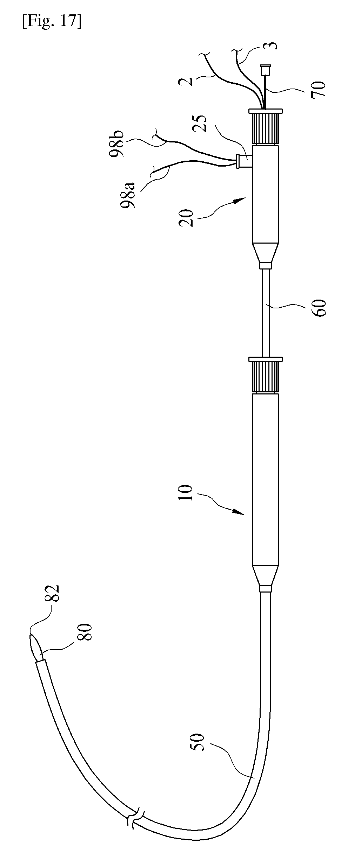

Referring to FIG. 17, the shape of a device for inserting a stent according to a third exemplary embodiment is illustrated. The device for inserting a stent according to the third exemplary embodiment is also the same as the device for inserting a stent according to the first exemplary embodiment in terms of overall configuration and operation and hereinafter, a difference therebetween will be primarily described.

In detail, two second guide wires 2 and 3 are inserted through the rear of the first aiding portion 20 in the third exemplary embodiment as illustrated in FIG. 17. In addition, two second guide wires 2 and 3 are both fixed to the first stent.

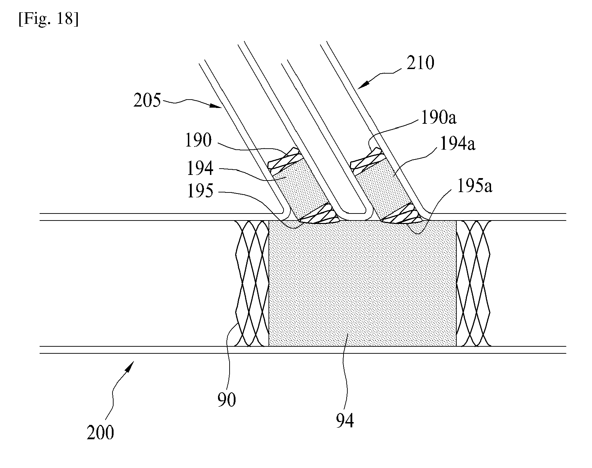

In addition, referring to FIG. 18, it can be seen that when two branch stenosis regions 205 and 210 are present in addition to the main stenosis region 200, the device for inserting a stent according to the third exemplary embodiment is used to install the stent at each stenosis region.

In this case, two second guide wires 2 and 3 illustrated in FIG. 17 are preferably fixed according to a distance between the branch stenosis regions 205 and 210 illustrated in FIG. 18, which are spaced apart from each other. Further, as illustrated in FIG. 18, when the blood vessel graft 94 is provided in the first stent 90, through-holes 195 and 195a may be formed as many as the second guide wires 2 and 3.

In addition, in the case of the third exemplary embodiment, a process is performed, which installs the first stent 90 and the second stent 190 at the main stenosis region 200 and the one branch stenosis region 205 through the same process as the first exemplary embodiment and thereafter, retreats the second outer tube inserted into the first outer tube to secure the inner space of the first outer tube for installing the second stent 190.

Thereafter, another second stent 190a may be installed by similarly repeating the process of installing the second stent 190 through another second guide wire 3.

In this case, the same process may be repeated by installing another second stent 190a at the second stent insertion unit used in the existing second guide wire 2 and inserting the second stent 190a into another second guide wire 3, but separate second stent insertion units are preferably used in the second guide wires 2 and 3, respectively. The reason is that stability of the surgical operation may be further secured only when the surgical operation is rapidly performed.

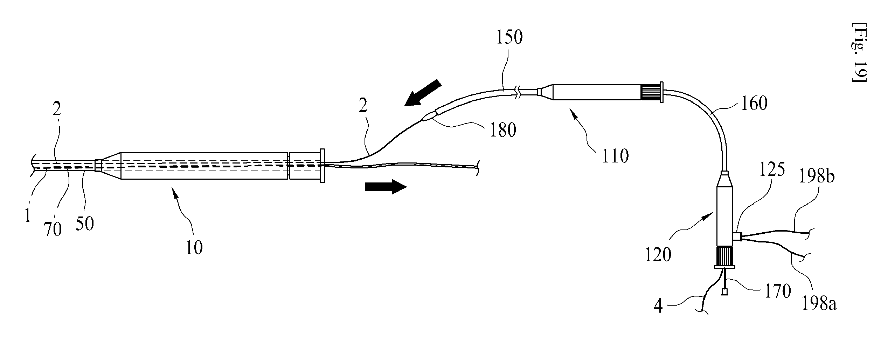

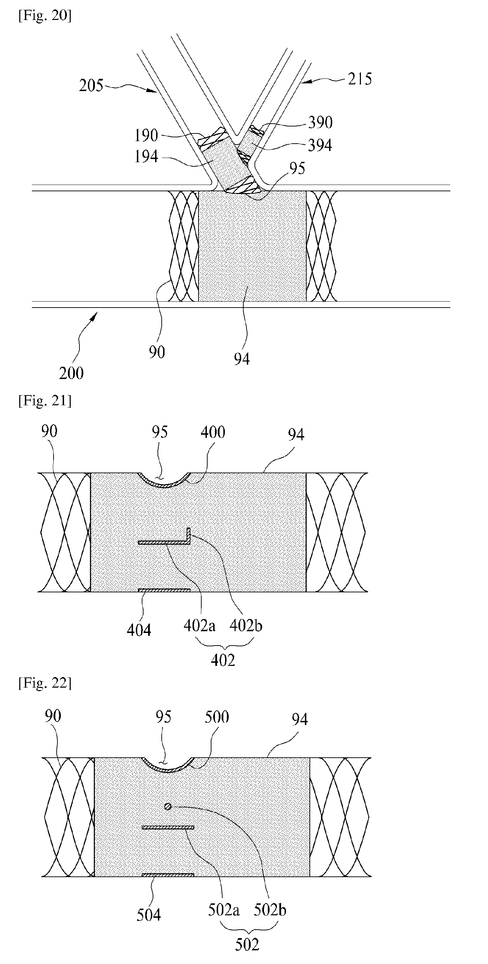

Next, a device for inserting a stent according to a fourth exemplary embodiment of the present invention and a surgical operation method using the same will be described with reference to FIGS. 19 and 20.

As illustrated in FIG. 19, the device for inserting a stent according to the fourth exemplary embodiment also generally has the same configuration and operation method as the device for inserting a stent according to the first exemplary embodiment. In addition, an illustrated shape shows the moment of inserting the second insertion unit into the second guide wire 2. In this case, in the case of the device for inserting a stent according to the fourth exemplary embodiment, it can be seen that a third guide wire 4 is inserted into the second insertion portion 110.

In detail, referring to FIG. 19, in respect to the second insertion portion 110, like the case in which the second guide wire 2 is inserted into the first insertion portion 10, the third guide wire 4 is inserted into the second insertion portion 110 and the third guide wire 4 is fixed to the second stent 190 provided at the front end of the second outer tube 150. In addition, a total of two fixation wires 198a and 198b are exposed to the second aiding portion 120 and this is to easily position the through-hole 95 of the blood vessel graft 194 of the second stent 190 at another branch stenosis region 215 in order to install a third stent 390 illustrated in FIG. 20 thereafter, similar to sequentially controlling the extension of the first stent 90 in the first exemplary embodiment.

That is, the device for inserting a stent according to the fourth exemplary embodiment may insert the stent into another branch stenosis region 215 branched from the branch stenosis region 205 in addition to the branch stenosis region 205 branched from the main stenosis region 200. This may be clearly different from other stent inserting devices contrived in the related art.

To this end, although not illustrated, the third stent insertion unit is further provided in the fourth exemplary embodiment and the third stent insertion unit includes a third insertion portion to which a third outer tube is extended and a third aiding portion to which a third inner tube is extended. Further, the third outer tube has a smaller diameter than the second outer tube 150 to be inserted into the second outer tube 150.

The third stent insertion unit is operated to insert the third stent 390 into another branch stenosis region 215 branched from the branch stenosis region 205.

Hereinabove, the first to fourth exemplary embodiments of the present invention have been described. In addition, it can be seen that the present invention is applied in addition the exemplary embodiments to be applied to various situations including the case in which a plurality of branch stenosis regions are present at the main stenosis region and the case in which another branch stenosis region is continuously branched from the branch stenosis region.

Meanwhile, in general, a surgical operator performs an operation of positioning each stent at a corresponding branch blood vessel outside through devices such as an X-ray, and the like while monitoring the operation. However, in this case, a displayed screen as a 2D screen projected by the X-ray, and the like, it is difficult to determine a rotational displacement of each stent by the displayed screen.

For example, when the first stent 90 of the first exemplary embodiment illustrated in FIG. 6 is described as an example, the first stent 90 needs to be rotated at the time of inserting the first stent 90 so that the through-hole 95 of the blood vessel graft 94 faces an inlet portion of the branch stenosis region 205 in order to position the second guide wire 2 at the branch stenosis region 205.

However, since the surgical operator monitors a 2D image, whether the through-hole 95 displayed in the screen is positioned at a close portion or a distant portion based on the screen may not be distinguished when the through-hole 95 illustrated in FIG. 6 does not face an inlet of the branch stenosis region 205. Accordingly, the surgical operator may not rapidly determine which direction the first stent 90 needs to be rotated in. Therefore, hereinafter, a method for solving the problem is presented.

FIG. 21 illustrates the shape of the first stent 90 with the blood vessel graft 94 for solving the problem in a device for inserting a stent according to a fifth exemplary embodiment of the present invention.

The first stent 90 includes a body in which wires cross each other, a plurality of holes are formed on the surface, a hollow is formed therein, and the circumference is formed to be extended and contracted, and the blood vessel graft 94 provided to encompass the circumference of the body and having the through-hole 95 formed at one side.

In addition, as illustrated in FIG. 21, it can be seen that markers 400, 402, and 404 are further provided in the blood vessel graft 94. In particular, a first marker 400 is provided on the circumference of the through-hole 95, a third marker 404 is provided at an opposite side of the through-hole 95, and a second marker 402 is provided at a middle portion between the first marker 400 and the third marker 404. In this case, the second marker 402 includes a reference marker 402a and a comparison marker 402b in detail.

In detail, the reference marker 402a and the comparison marker 402b are formed to recognize the rotational direction of the first stent 90 by comparing relative positions and in the case of the fifth exemplary embodiment, the reference marker 402a elongates in the longitudinal direction of the first stent 90 and the comparison marker 402b is bent from the reference marker 402a to be extended in the width direction of the first stent 90.

That is, the second marker 402 serves to verify the position of the through-hole 95 to recognize which direction the first stent 90 needs to be rotated in even through the 2D image.

In detail, when the through-hole 95 is positioned at the close portion based on the screen, the second marker 402 is bent upward and when the through-hole 95 is positioned at the distant portion based on the screen, the second marker 402 is bent downward. Accordingly, the surgical operator may rapidly determine which direction the first stent 90 needs to be rotated in.

In addition, the rotational displacement of the first stent 90 is corrected through the second marker 402 and thereafter, a horizontal position of the first stent 90 is slightly offset, and as a result, the first stent 90 may need to slightly move back and forth. In this case, the first marker 400 and the third marker 404 are positioned at upper and lower sides of the screen, respectively based on the first marker 400 and the third marker 404 and the rotational displacement may be prevented from being offset again by moving the first stent 90.

As described above, the device for inserting a stent according to the fifth exemplary embodiment is advantageous in elaborately perform the surgical operation while monitoring the 2D screen.

Next, referring to FIG. 22, in a device for inserting a stent according to a sixth exemplary embodiment of the present invention, the shape of the first stent 90 is illustrated. It can be seen that a first marker 500 and a third marker 504 of the first stent 90 in the sixth exemplary embodiment are formed similarly to those in the fifth exemplary embodiment, but a form of a second marker 502 is different.

In detail, a reference marker 502a elongates in the longitudinal direction of the first stent 90, a comparison market 502b is formed in a point shape, and the comparison marker 502b is positioned to be spaced apart from the reference marker 502a in the width direction of the first stent 90.

That is, even in this case, relative positions of the reference marker 502a and the comparison marker 502b are determined to determine the rotational direction of the first stent 90 even in the 2D image.

Consequently, the shape of the second marker 502 may be diversified, however, if the relative positions of the reference marker 502a and the comparison marker 502b are determined to determine the rotational direction of the first stent 90, there is no limit in the shape of the second marker 502.

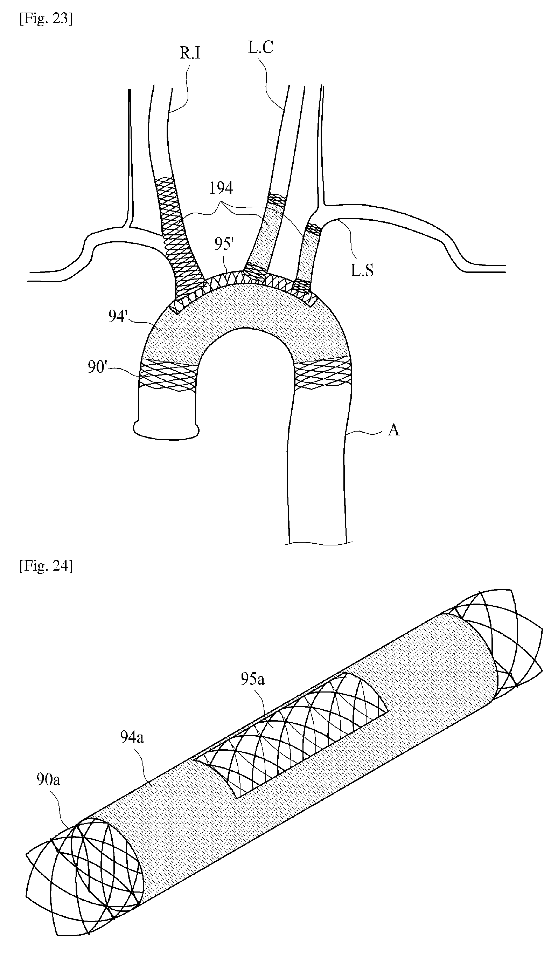

FIG. 23 is a perspective view illustrating the shape of a first stent in a device for inserting a stent according to a seventh exemplary embodiment of the present invention. FIG. 24 is a diagram illustrating a state in which the first stent is installed at an ascending aorta in the device for inserting a stent according to the seventh exemplary embodiment of the present invention.

Referring to FIGS. 23 and 24, an opening hole 95a of a first stent 90a of the exemplary embodiment is widely formed in a retraction type unlike the above embodiment. Therefore, the opening hole 95 of the first stent 90 in the exemplary embodiment is formed to correspond to the branch blood vessel, while the opening hole 95 has a size to include inlets of one or more branch blood vessels.

Accordingly, when lesions generally occur at a point adjacent to one or more branch blood vessels formed in the human body, the surgical may be rapidly performed without preventing a blood flow from flowing into a plurality of branch blood vessels.

Meanwhile, the first stent 90a in the exemplary embodiment may be installed at various points, but in particular, when the first stent 90a is installed at the ascending aorta, an effective surgical operation may be performed.

In detail, a right innominate artery (R.I.), a left common carotid artery (L.C.), and a left subclavian artery (L.S.) are disposed to be adjacent to an aortic arch of the ascending aorta. Accordingly, when lesions such as aortic dissection, and the like occurs at the ascending aorta, the first stent 90a needs to be surgically operated at the ascending aorta without preventing the blood flow from flowing into the branch blood vessels.

In particular, if the lesions at the aorta are not rapidly surgically operated, a life may be threatened, and as a result, the rapid surgical operation is further required.

Therefore, the opening hole 95a in the exemplary embodiment has the size to include the inlets of one or more branch blood vessels. In addition, the surgical operator performs a stent inserting operation while verifying the position of the opening hole 95a through the 2D image as described in the exemplary embodiment.

Meanwhile, like the above exemplary embodiment, even in the first stent 90a of the exemplary embodiment, markers having various shapes including a circumference portion of the opening hole 95a, and the like, which allow the surgical operator to verify the position of the opening hole 95a may be provided at an blood vessel graft 94a.

Meanwhile, the opening hole 95a in the exemplary embodiment has a quadrangular shape and is formed to be opened at the blood vessel graft 94a. However, the opening hole 95a may have the size and the shape to include the plurality of branch blood vessels.

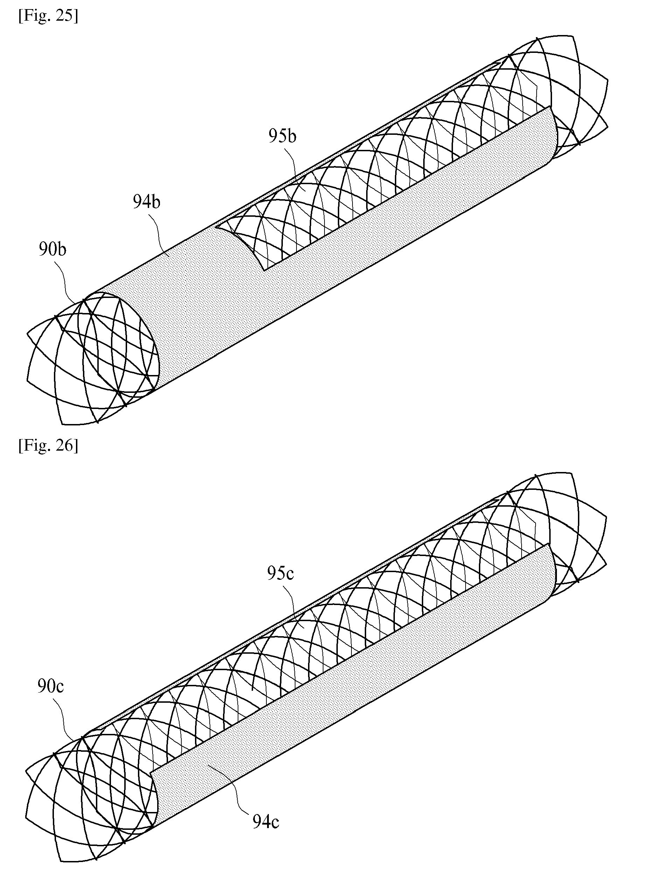

For example, like a first stent 90b illustrated in FIG. 25, an opening hole 95b may be formed in communication with one end of an blood vessel graft 94b. In addition, as illustrated in FIG. 26, in an opening hole 95c, a first stent 90c may be formed in communication with both ends of an blood vessel graft 94c.

Further, the second stent 194 having various forms may be installed at each branch blood vessel through the opening hole 95a and the installation process is the same as that of the exemplary embodiment, and as a result, a description thereof will be omitted.

Although the specific exemplary embodiments have been described and illustrated as above, the present invention is not limited to the exemplary embodiments described herein, and it would be apparent to those skilled in the art that various changes and modifications might be made to these exemplary embodiments without departing from the spirit and the scope of the invention. Accordingly, the changed example and modified examples should not be individually appreciated from the technical spirit or the viewpoint of the present invention and it should be appreciated that modified exemplary embodiments will be included in the appended claims of the present invention.

## Claims

1. A device for inserting a stent, the device comprising:
a first stent insertion unit including a first insertion portion formed by extending a first outer tube, a first aiding portion formed by extending a first inner tube inserted into the first insertion portion to be slid back and forth inside the first outer tube, a first central tube into which a first guide wire is to be inserted, and which is inserted into the first aiding portion to penetrate the first inner tube, and a first stent inserted into the front end of the first outer tube while encompassing the circumference of the first central tube and discharged outside the first outer tube by forward movement of the first inner tube so as to be extended; and
a second stent insertion unit including a second insertion portion formed by extending a second outer tube to be inserted into the first outer tube, a second aiding portion formed by extending a second inner tube inserted into the second insertion portion to be slid back and forth inside the second outer tube, a second central tube which is inserted into the first insertion portion to be extended between the first outer tube and the first inner tube, into which a second guide wire fixed to the first stent is inserted, and which is inserted into the second aiding portion to penetrate the second inner tube, and a second stent inserted into the front end of the second outer tube while encompassing the circumference of the second central tube and discharged outside the second outer tube by forward movement of the second inner tube so as to be extended.

2. The device of claim 1, wherein one or more extension preventing members that prevent extension of the stent are provided on the circumference of at least one of the first stent and the second stent, and
fixation wires are further provided, which fix the extension preventing members and are extended through the corresponding inner tubes to protrude outside the corresponding aiding portions.

3. The device of claim 1, wherein a guide member is provided at the front end of at least one of the first central tube and the second central tube, in which the guide member protrudes outside each of the corresponding outer tubes and has a larger diameter than each of the corresponding outer tubes.

4. The device of claim 1, wherein the second guide wire is inserted into the inside of the first insertion portion through the rear of the first aiding portion.

5. The device of claim 1, wherein the second guide wires are provided as many as stents to be installed.

6. The device of claim 1, wherein an blood vessel graft is provided on the circumference of at least one of the first stent and the second stent, and
a through-hole through which the second guide wire passes is formed at the blood vessel graft provided in the first stent.

7. The device of claim 1, wherein a fixation member that fixes the first inner tube is provided at the first insertion portion, in a part where the first inner tube is inserted into the inside of the first insertion portion.

8. The device of claim 1, further comprising:
a third stent insertion unit including a third insertion portion formed by extending a third outer tube inserted into the second outer tube, a third aiding portion formed by extending a third inner tube inserted into the third insertion portion to be slid back and forth inside the third outer tube, a third central tube which is inserted into the third insertion portion to be extended between the second outer tube and the second inner tube, into which a third guide wire fixed to the second stent is inserted, and which is inserted into the third aiding portion to penetrate the third inner tube, and a third stent inserted into the front end of the third outer tube while encompassing the circumference of the third central tube and discharged outside the third outer tube by forward movement of the third inner tube so as to be extended.

9. A device for inserting a stent, the device comprising:
a first stent insertion unit including a first insertion portion formed by extending a first outer tube, a first aiding portion formed by extending a first inner tube inserted into the first insertion portion to be slid back and forth inside the first outer tube, a first central tube into which a first guide wire is to be inserted, and which is inserted into the first aiding portion to penetrate the first inner tube, and a first stent inserted into the front end of the first outer tube while encompassing the circumference of the first central tube and discharged outside the first outer tube by forward movement of the first inner tube so as to be extended; and
a second stent insertion unit including a second insertion portion formed by extending a second outer tube to be inserted into the first outer tube, a second aiding portion formed by extending a second inner tube inserted into the second insertion portion to be slid back and forth inside the second outer tube, a second central tube which is inserted into the first insertion portion to be extended between the first outer tube and the first inner tube, into which a guide tube fixed to the first stent is inserted so as to insert a second guide wire, and which is inserted into the second aiding portion to penetrate the second inner tube, and a second stent inserted into the front end of the second outer tube while encompassing the circumference of the second central tube and discharged outside the second outer tube by forward movement of the second inner tube so as to be extended.

10. The device of claim 9, wherein one or more extension preventing members that prevent extension of the stent are provided on the circumference of at least one of the first stent and the second stent, and
fixation wires are further provided, which fix the extension preventing members and are extended through the corresponding inner tubes to protrude outside the corresponding aiding portions.

11. The device of claim 9, wherein a guide member is provided at the front end of at least one of the first central tube and the second central tube, in which the guide member protrudes outside each of the corresponding outer tubes and has a larger diameter than each of the corresponding outer tubes.

12. The device of claim 9, wherein the guide tube is inserted into the inside of the first insertion portion through the rear of the first aiding portion.

13. The device of claim 9, wherein the guide tubes are provided as many as stents to be installed.

14. The device of claim 9, wherein an blood vessel graft is provided on the circumference of at least one of the first stent and the second stent, and
a through-hole through which the guide tube passes is formed at the blood vessel graft provided in the first stent.

15. The device of claim 9, wherein a fixation member that fixes the first inner tube is provided at the first insertion portion, in a part where the first inner tube is inserted into the inside of the first insertion portion.

16. A surgical operation method for inserting a stent in the device for inserting a stent of any one of claims 1 to 8, the method comprising:
inserting a first guide wire into a main stenosis region;
inserting the first guide wire into a first central tube to insert a first outer tube into the main stenosis region along the first guide wire;
operating a first stent insertion unit to move the first inner tube forward, thereby discharging a first stent to the main stenosis region and placing the front end of a second guide wire at a branch stenosis region;
extending the first stent;
retreating a first inner tube to secure an inner space of the first outer tube;
inserting the back end of the second guide wire into a second central tube to insert a second outer tube into the branch stenosis region along the second guide wire;
operating the second stent insertion unit to move the second inner tube forward, thereby discharging the second stent to the branch stenosis region; and
extending the second stent.

17. The method of claim 16, wherein one or more extension preventing members that prevent extension of the stent are provided on the circumference of at least one of the first stent and the second stent,
fixation wires are provided, which fix the extension preventing members and are extended through each corresponding inner tube to protrude outside each corresponding aiding portion, and
at least one of the extending of the first stent and the extending of the second stent includes removing the fixation wire from the extension preventing member.

18. The method of claim 16, wherein a plurality of second guide wires are provided, and after the extending of the second stent,
retreating the second outer tube to secure an inner space of the first outer tube, inserting the rear end of another second guide wire into the second stent insertion unit or a second central tube of another second stent insertion unit to insert a second outer tube into another branch stenosis region along another second guide wire, operating the second stent insertion unit to move the second inner tube forward, thereby discharging another second stent to another branch stenosis region, and extending another second stent are repeatedly performed as many as the second guide wires.

19. The method of claim 16, wherein a fixation member that fixes the first inner tube is provided at the first insertion portion, in a part where the first inner tube is inserted into the inside of the first insertion portion, and
fixing the first inner tube with the fixation member is further included between the securing of the inner space of the first outer tube and the inserting of the second outer tube into the branch stenosis region.

20. A surgical operation method for inserting a stent in a device for inserting a stent of any one of claims 9 to 15, the method comprising:
inserting a first guide wire into a main stenosis region;
inserting the first guide wire into a first central tube to insert a first outer tube into the main stenosis region along the first guide wire;
operating a first stent insertion unit to move the first inner tube forward, thereby discharging a first stent to the main stenosis region and placing the front end of a second guide wire at a branch stenosis region;
extending the first stent;
inserting the second guide wire into the guide tube;
retreating the first inner tube and the guide wire to secure an inner space of the first outer tube;
inserting the back end of the second guide wire into a second central tube to insert a second outer tube into the branch stenosis region along the second guide wire;
operating the second stent insertion unit to move the second inner tube forward, thereby discharging the second stent to the branch stenosis region; and
extending the second stent.

21. The method of claim 20, wherein one or more extension preventing members that prevent extension of the stent are provided on the circumference of at least one of the first stent and the second stent and
fixation wires are provided, which fix the extension preventing members and are extended through each inner tube to protrude outside each corresponding aiding portion, and
at least one of the extending of the first stent and the extending of the second stent includes removing the fixation wire from the extension preventing member.

22. The method of claim 20, wherein a fixation member that fixes the first inner tube is provided at the first insertion portion, in a part where the first inner tube is inserted into the inside of the first insertion portion, and
fixing the first inner tube with the fixation member is further included between the securing of the inner space of the first outer tube and the inserting of the second outer tube into the branch stenosis region.

23. A stent comprising:
a body in which a plurality of holes are formed on the surface, a hollow is formed inside by intersecting wires, and a circumference is extensible and contractable; and
an blood vessel graft provided to encompass the circumference of the body, has a through-hole at one side, and guides the displacement of the body.

24. The method of claim 23, wherein the marker includes:
a first marker formed on the circumference of the through-hole;
a third marker formed on the opposite circumference of the through-hole; and
a second marker including a reference marker formed between the first marker and the third marker, and the reference and a comparison marker formed between the first marker and the third marker and allowing a rotational direction of the body to be recognized through comparison with each other.

25. A stent installed in a blood vessel with a plurality of branch blood vessels, the stent comprising:
a body in which a plurality of holes are formed on the surface, a hollow is formed inside by intersecting wires, and a circumference is extensible and contractable; and
an blood vessel graft provided to encompass the circumference of the body and having a through-hole at one side, in which the through-hole has a size to include inlets of the plurality of branch blood vessel.
